# EUROPEAN PATENT APPLICATION

(11) **EP 2 065 049 A1**
(43) Date of publication of application: **03.06.2009**
(21) Application number: 07763992.0
(22) Date of filing: 06.07.2007
(51) Int. Cl.: A61K 36/28, A61P 31/12, A61P 31/16, A61P 31/18

(54) **THE USE OF SOLIDAGO VIRGAUREA IN THE PREPARATION OF A MEDICAMENT FOR TREATMENT AND PREVENTION OF A VIRUS**

(30) Priority: 07.09.2006 CN 200610127150; 07.09.2006 CN 200610127149
(71) Applicant: Lou, Shirong, 310009 Hangzhou City Zhejiang Province (CN)
(72) Inventor: Lou, Shirong, 310009 Hangzhou City Zhejiang Province (CN)
(74) Representative: Hryszkiewicz, Danuta
(86) International application number: PCT/CN2007/002084
(87) International publication number: WO 2008/031315

(57) **Abstract**

The use of Solidago virgaurea in the preparation of a medicament for treatment and prevention of a virus, in particular a H5N1"bird flu" virus, an influenza virus or an AIDS virus.

## Description

This invention relates to use of Solidago virgaurea in the treatment and prevention of viral infections and the lowering of the concentration of viruses in an animal, e.g., a human. More specifically this invention relates to the use of Solidago virgaurea in the treatment and prevention of the H5N1 avian influenza and to lowering of the concentration of the H5N1 avian influenza virus in an animal, e.g., a human. This invention also relates to the use of Solidago virgaurea in the treatment and prevention of AIDS and to lowering of the concentration of HIV in an animal, e.g., a human.

Influenza A virus subtype H5N1, also known as A (H5N1) or simply H5N1, is a subtype of the Influenza A virus which can cause illness in humans and many other animal species. Influenza is a commonly occurring disease that has not been eliminated heretofore, and the difficulty of treating influenza lies in the ongoing mutations of the virus. Because humans are facing the threat of mutation of H5N1 highly pathogenic avian influenza, there is an urgent need to develop effective drugs that can stop influenza virus ongoing mutations and that of various other types of influenza viruses.

It has been only about 20 years since the discovery of AIDS, yet the high lethality and virulence of HIV has resulted in infections of over 38 million people and has caused death of over 25 million people worldwide. At present, there are more than ten thousand of new persons infected by AIDS virus daily. Even though the medical profession and the governments throughout the world are doing their best to study and prevent AIDS, so far no effective cure for HIV/AIDS have been found. The common opinion of the AIDS experts at the 16th World HIV/AIDS Assembly was that the scientific research field of AIDS lacks an essential breakthrough. Therefore, the development of effective drugs for treating and preventing HIV/AIDS continues to be an urgent priority.

According to pharmacopoeia records, the use of Solidago virgaurea in the traditional Chinese medicine is mainly to dispel wind, remove heat, subdue swelling, detoxicate, treat coldness and headache, sore throat, jaundice, pertussis, children infantile convulsion, traumatic injury, sores of the back, and tinea manuum. Moreover, Solidago virgaurea has apparent bacteriostatic and bacteriocidal properties with respect to staphylococcus aureus, pneumococcus, pseudomonas aeruginosa, shigella flexneri, etc.

In view of the above-described problems, it is one objective of the invention to provide a new use of Solidago virgaurea pure plant herb in the preparation of a medicament for treatment and prevention of virus.

To achieve the above objectives, in accordance with one embodiment of the invention, provided is a medicament, comprising Solidago virgaurea and a use of Solidago virgaurea in the preparation of the medicament for treatment and prevention of H5N1 avian influenza virus or influenza virus based on the basic principle of Traditional Chinese Medicine.

This invention provides a use of Solidago virgaurea in the preparation of a medicament for treatment and prevention of HIV on the basic principle of Traditional Chinese Medicine.

The active ingredient of the medicament for treatment and prevention of virus may comprise 50-100% Solidago virgaurea by weight.

The virus may be H5N1 avian influenza, influenza virus or HIV.

The medicament may comprise the sole the active ingredient Solidago virgaurea. For example, the Solidago virgaurea is mixed with water in a weight ratio of 1:50-100 into an extractor, boiled for 10-30 minutes, filtered and purified with conventional methods to obtain an herbal stock solution.

Alternatively, the Solidago virgaurea is mixed with alcohol in a weight ratio of 1:50-100 into an extractor, boiled for 10-30 minutes, filtered and purified with conventional methods to obtain an herbal stock solution.

The medicament may be provided in the form of an oral liquid, a granule, a decoction piece, a capsule, spray, a pill, an injection, or freeze dried powder.

Solidago virgaurea used herein may be the entire Solidago virgaurea plant or parts thereof.

The active ingredients of the medicament may comprise 50-90% Solidago virgaurea and 10-50% glycyrrhiza by weight.

The active ingredients of the medicament may comprise 50-90% Solidago virgaurea, 5-25% honeysuckle, and 5-25% radix isatidis by weight.

In another respect, provided is a use of Solidago virgaurea in the preparation of a medicament for treatment and prevention of a virus. The medicament is provided in form of a granule, a capsule or an herb decoction prepared using micron-milling technology, or in form of tea packages prepared by milling, and the weight ratio of Solidago virgaurea with respect to the entire composition is 50-100%.

Alternatively, the active ingredients of the medicament may comprise 50-90% Solidago virgaurea and 10-50% glycyrrhiza by weight.

Alternatively, the active ingredients of the medicament may comprise 50-90% Solidago virgaurea, 5-25% honeysuckle and 5-25% radix isatidis by weight.

The medicament of the invention may be provided in the form of an injection preparation, freeze dried powder, decoction, oral liquid, granule, spray, capsule or dropping pill. Solidago virgaurea may be provided in form of an extract by supercritical CO₂, and the weight ratio of Solidago virgaurea in the medicament is 50-100%.

Conventional purification methods of Solidago virgaurea are as follows.

(1) The common apparatus for the purification of technology of the water extract: sedimentation tank, centrifuge, ultrafilter and ultrafiltration membrane etc.

(2) Technology process: According to the theory that solid particles can be separated from liquids in the liquid medium by natural sedimentation due to the gravity, in the sedimentation separation technology, put the water extract into the sedimentation tank, let it stand for a period of time, and then remove the upper liquid using a siphon. Also, temperature reduction and the addition of sedimentation agent - electrolyte (such as alum) can be used to achieve higher efficiency and higher quality if necessary.

The existing micron-milling wall-braking technique is as follows:

It is completed by use of a device called "air flow pulverizer" which combines the nanometer milling and wall-braking technologies developed by Angli Natural Drug Engineering Technology Ltd. of Shanghai Jiaotong University.

The theory is that after the materials enter the milling room of air flow pulverizer in which there are several milling nozzles, the supersonic flow of the milling nozzles gets the milled materials impacted by the high-speed air flow and the mutual collision among the powders, and due to the high speed air flow and strong impact force, the milling effect can reach 0.5-10 micron-size particles (500 nm-1 0000 nm) and realize physical plant cell wall breaking to make the effective materials completely available for the human body to absorb.

The existing supercritical CO₂ liquid extraction technique is as follows:

The extraction is completed using "1000 L automatic large scale supercritical units" developed by Shenyang DongYu Corp. with high extraction capability, high extraction rate and controllable quality.

The theory is that the critical temperature and pressure of CO₂ are respectively 31.05°C and 7.38 MPa, and CO₂ has double characteristics of a gas and a liquid when above the critical point. It is similar to a gas as it has a similar viscosity to gas; as well it is similar to a liquid as it has a similar density to liquid; however, it has a much larger diffusion coefficient than liquid. Meanwhile, it is a good solvent, and can dissolve many materials by the mutual function and diffusion function among the molecules. At the same time, in the regions higher than the critical point, pressure has a little change, which can cause great changes of its density so as to bring larger changes of the solubility. Therefore, supercritical CO₂ can dissolve the materials in the matrix, forming the supercritical CO₂ load phase, then reducing the pressure of the carrier gas or rising temperature, the solubility of supercritical CO₂ is reduced, and these materials are separated from CO₂ (resolution) so as to realize the extraction and separation goal.

The use and effective dose of the medicament of this invention for treating H5N1 avian influenza or influenza virus or treating AIDS virus are listed as follows:

The oral dose of the herbal stock solution is 2-4 times per day and 100-300 ml per time for adults.

For the oral liquid, the oral dose is 2-4 times per day and 10-20 ml per time for adults.

For the granule, capsules or decoction pieces, the dose is 3-4 times per day and 2-6 g per time for adults.

For the injection, the dose is twice per day and 5-10 ml per time for adults.

For the dropping pill, the dose is three times per day and 8 pills per time for adults.

For the spray sprayed in the mouth, the dose is several times per day.

The use and effective dose of the medicament for preventing H5N1 avian influenza or influenza virus or preventing AIDS virus of this invention are the same as above.

According to the records in page 8 to page 9 of the Dictionary of Traditional Chinese drugs (first volume, edited by Jiangsu New College of Medicine), the Solidago virgaurea is a composite plant; its whole herb or the whole herb with root are usually used; it is bitter and cool taste. The whole herb Solidago virgaurea contains hydroxybenzene ingredient, tannin, volatile oil, saponin, and flavonoids, etc. Its main functions are mainly in dispelling wind and cleaning heat, subduing swelling and detoxication, treating cold and headache, sore throat, jaundice, pertussis, children infantile convulsion, traumatic injury, carbuncle on the back and tinea manuum. Moreover, it has obvious killing and inhibition functions on staphylococcus aureus, pneumococcus, pseudomonas aeruginosa and shigella flexneri, etc.

The medical efficiency of Solidago virgaurea is in dispelling wind and cleaning heat, diminishing inflammation and detoxicating; its pharmacological functions can reach liver and gallbladder meridians with cool medical property.

Glycyrrhiza can purge fire, detoxify and moisten lung to stop coughing; its pharmacological functions can reach all the twelve meridians; it has mild medical nature and can be in concordance with other drugs.

The medical efficiency of honeysuckle is in cleaning heat and detoxicating, diminishing inflammation and alleviating pain; its pharmacology functions into lung, stomach and colorectal meridians; and cold medical property.

The medical efficiency of radix isatidis is in cooling blood and clearing fire, diminishing inflammation and analgesia; its pharmacology functions into heart and lung meridians; and cold medical property.

The combination of Solidago virgaurea and glycyrrhiza can regulate the medical property of Solidago virgaurea and enhance the adaptability.

The combination of Solidago virgaurea with honeysuckle and radix isatidis can improve the attending efficiency and increase the broad-spectrum property.

Experiments are further provided to prove the effect of Solidago virgaurea in use for preparing drugs for treating and preventing H5N1 avian influenza or influenza virus.

With decades of clinical experiences, the invention validates that the herbal stock solution not only has obvious killing and inhibition functions on H5N1 avian influenza or influenza virus, but also has very good variation resistance.

This invented medicament is used for treating and preventing H5N1 avian influenza or influenza virus, and the efficiency rate is above 90%.

The following statistical reports of clinical observation in curative effect are used to further validate the effects of this invention.

1. Clinical Data:

Treating and preventing various types of influenza

1) Using Solidago virgaurea extract with concentration of 1:100 to treat 513 cases with influenza A, 2-4 times of oral administrations for patients, 300 mL per time for adults and about 20-300 mL for children according to their ages. Results had shown that 399 cases were cured within one day, accounting for 77.8%; 92 cases were cured within two days, accounting for 17.9%; 22 cases were cured within three days, accounting for 4.3%. The total effective rate was 100%.

2) Using Solidago virgaurea extract with concentration of 1:100 to treat 253 cases with influenza B, 2-4 times of oral administrations for patients, 300 mL per time for adults and about 20-300 mL for children according to the age. Results had shown that 165 cases were cured within one day, accounting for 65.2%; 46 cases were cured within two days, accounting for 18.2%; 25 cases were cured within three days, accounting for 9.9%; 17 cases were inefficient, accounting for 6.7%. The total effective rate was 93.3 %.

3) Using Solidago virgaurea extract with concentration of 1:100 to treat 116 cases with influenza C, 2-4 times of oral administrations for patients, 300 ml per time for adults and about 20-300 ml for children according to the age. Results had shown that 51 cases were cured within one day, accounting for 44%; 27 cases were cured within two days, accounting for 23.3%; 28 cases were cured within three days, accounting for 24.1%; 10 cases were inefficient, accounting for 8.6%. The total effective rate was 91.4 %.

4) During the period of influenza, 1291 healthy persons took Solidago virgaurea extraction with the concentration of 1:100 to prevent the influenza virus infection, one time per day, 60-150 ml for adults and 20-80 ml for children. Results had proved that the effective prevention rate was 100%.

Treating undefined pneumonia

On December 2003 and September 2004, 2 cases with undefined pneumonia were cured using Solidago virgaurea extract with concentration of 1:100. The patients were both adults; they were the patients in ICU in Zhejiang Tongde Hospital and the First Affiliated Hospital of Zhejiang Medical University, respectively; and both had sustaining hyperpyrexia (above 39°C) for more than seven days with rapid disease development, appearing pulmonary hemorrhage, pleural effusions, dyspnea and pulmonary signs of consolidation; SARS virus infection was excluded with laboratory RNA and PCR detections, however, virus could be extracted from tracheal exsuction materials of the patients; the chest image detected serious lung infusion, showing consolidation image of lungs with large ground-glass shape. Both hospitals sent "Notice of Critical Illness". Under this condition, patient relatives asked for help from the inventor. Solidago virgaurea extract was used with the concentration of 1:100 to treat the two patients, 3-4 times per day, 300-500 mL per time and sequential administration for three days. The body temperature of the patients reduced to below 37.8°C within two days with rapid spirit recovery; the life signs obviously became better, and then entered the pneumonia recovery period in the common sickroom. They left hospital after about 15 days with good prognosis.

Treating and preventing H5N1 avian influenza.

1. On 13 January 2004, a large number of chickens died in the farm in Dongting village, Guangde County, Anhui province. Using Solidago virgaurea extract with the concentration of 1:60 to cure 584 infected chickens from 16 January, 40-60 mL of oral dose for each chicken, adding another administration after two hours; feeding 242 healthy chicken respectively with the fine feedstuff mixed in a ratio of 1:4 with Solidago virgaurea extract with the concentration of 1:100, and with the fine feedstuff mixed in a ratio of 1:10 with Solidago virgaurea powder. According to the observation results after two hours, four hours, ten hours, 30 hours, 60 hours and five days, except 26 chicken died during initial administration period (within five minutes), other chicken all recovered with normal activities; the effective rate was above 95%; all healthy chicken were protected efficiently, and protection effective rate was up to 100%. The participating workers and chicken farm workers drank a cup of 300 mL of Solidago virgaurea extract with the concentration of 1:100 to prevent virus infection, and they all retain health at the end of experiment (At the beginning of Feb, the National Disease Controlling Center determined that the epidemic broken out in Guangde country of Anhui was H5N1 highly pathogenic avian influenza).

2. The diagnosis is based on GB 15994-1995 Diagnosis Criteria for Influenza and GB/T18936-2003 Diagnosis Technique for Highly Pathogenic Avian Influenza.

3. Treatment method: for treating influenza: 10 g of Solidago virgaurea is boiled in 1000 mL of water for ten minutes, the obtained 900 mL of herbal stock solution in this invention is taken by patients; 2-4 times per day, and 300 mL per time. For treating avian influenza, 10 g of Solidago virgaurea is boiled in 600 mL of water, oral administration for 2-4 times per day, 40-60 mL per time.

4. The evaluation standards for curative effect: 1) patients feel better: body temperature reduces to below 37.5°C, dry cough is alleviated, with smooth breath; Cure: body temperature changes to normal, dry cough disappears; pneumonia recovers well, with normal breath. 2) The spirits of ill avian get well, with normal activities.

5. Curative effect observation: After the administration for three days, the spirit and mood of patients improved, with normal body temperature and a good prognosis; finally the patients were completely cured. The ill birds were in good spirits and had good appetites after five-day observation.

6. Conclusion: using the herbal in this invention to treat and prevent H5N1 influenza or influenza virus, the effective rate is above 90%.

Experiments are further provided to prove the effects of Solidago virgaurea in use for preparing drugs for treating and preventing HIV virus.

By the studies of inventor for several years and the validation of the laboratory in AIDS Research Center of Tropical Medicine Institute in Guangzhou University of TCM in China, the herbal stock solution in this invention had obvious HIV virus resistance.

According to double-blind requirements in the invention, Solidago virgaurea is mixed with water in the ratio of 1:120 to prepare two samples of HH2A (decoction over ten minutes) and HH2B (decoction over thirty minutes) with very low concentrations according to the decocting extraction concentration time. The two samples were sent to AIDS Research Center of Tropical Medicine Institute in Guangzhou University of TCM in China for the pharmacodynamics studies of AIDS virus strain called SIV virus.

The test report of the Anti-AIDS virus strain SIV in vitro is shown as follows:

Inspection Date: March 24 , 2006

Reporting Date: May 25 , 2006

Delivery Unit: 17-3-1-601 in Caihe east region of Hangzhou city, Zhejiang province

Delivery Specimens: medical solutions HH2A and HH2B.

Delivery Objective: to detect the activity of the anti- SIVmac cuvette of the delivery medical solution specimens via SIVmac-CEM×174 system.

I. Materials:

1. The cell lines: CEM×174 is from USAAarond Diamond AIDS Research Center, and is presented by Beijing Medical Laboratory Animal Institute.

2. The virus strains: SIVmac is from USAAarond Diamond AIDS Research Center, and is presented by Beijing Medical Laboratory Animal Institute.

3. The cell culture fluid: PRMI1640 culture fluid containing 10% of calf serum.

4. The pending detection samples: medical solutions HH2A and HH2B.

5. The positive control drug: AZT produced by CALBIOCHEM, and is for the experiment use especially.

6. The rhesus monkey IgG fluorescence: produced by E.Y.

7. The anti SIV monkey positive serum: the serum of the SIV infected monkeys in the recovery period.

8. Others: 96-hole cell culture plate and 24-hole cell culture plate.

II. Experimental Methods:

1. The toxicity experiment of the samples to be detected on CEM×174 cell lines:

1) Dilution of the samples to be detected: on the basis of the original concentration of the samples to be detected, diluting the samples 10 times with RPMI1640 culture fluid containing 10% of calf serum before the experiment.

2) Toxicity determination on CEM×174 cells: the samples were diluted with serial twice-dilution from the 1:10 diluted solution, those are 1:10, 1:20, 1:40, 1:80, 1:160, 1:320, 1:640 and 1:1280. The diluted samples to be detected were put into 96-hole cell culture plate, with 100 µl per hole. 3.0×10⁵/mL CEM×174 cells were added, with 100 µL per hole. The cells were cultured in an incubator containing 5% CO₂ and with saturated humidity at 37°C, and then the results were observed after four days.

3) The results of the toxicity experiments determination (See Tab. 1).

The toxicity determination basis: taking cell death and growth conditions as the determination basis, ++++, +++, ++, + and - represent as follows, respectively:

++++: The grown cells all died;

+++: Most of the grown cells died and a few of them had temporary splitting;

++: The proliferated cells were about 50% less than cell control holes, wherein there were many dead cells;

+: The proliferated cells were about 25% less than cell control holes;

-: The cells grew well and had no obvious differences compared with the cell control holes.

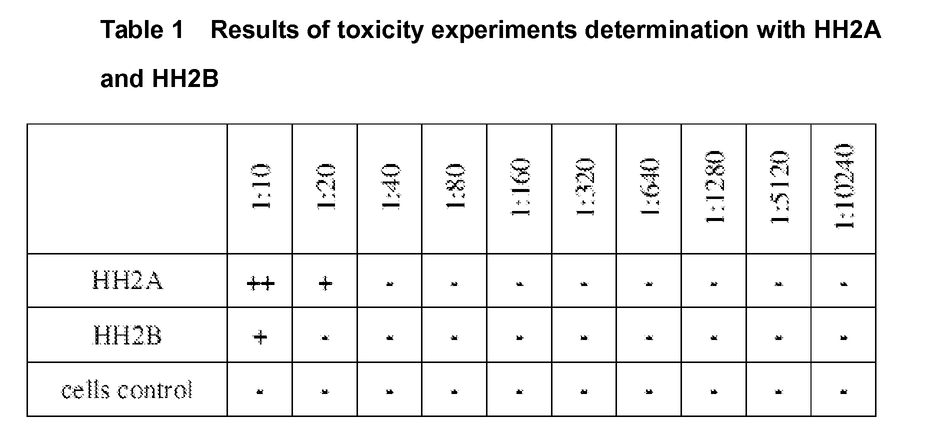

2. The formal experiment: done by 24-hole plate, and double holes for each item. The experiment is repeated twice.

(1) Diluting the drug using a series of nontoxic concentration, diluting the virus according to the experiment requirements, configuring the cells to be 3×10⁵ /ml cell suspension, adding various reagents (drugs, virus, cells, and culture fluids) to the holes by the listed order in Table. **2**, in addition setting AZT positive drug and SIV control hole. The cells were cultured in an incubator containing 5% CO₂ and with saturated humidity at 37°C. The medical solution with original dilution was updated once every three days. The results were determined when CPE of the SIV control hole showed +++ - ++++ after six to seven days.

(2) Firstly, observe CPE of the experimental plate: Culture the supernatant fluid to determine the titer of virus through serial twice-dilution, and calculate the descending amounts of the virus yield; wash the other cells with PBS, smear, and then calculate the fluorescence positive cell amounts by using the indirect immunofluorescence method.

**Tab. 2 The addition of reagents of each hole in formal experiment**

| | Drug hole | AZT positive control hole | SIV control hole |
|---|---|---|---|
| Drugs to be detected | 0.8 mL | - | - |
| AZT (2µM) | - | 0.8 mL | - |
| SIV (10TCID50) | 0.4 mL | 0.4 mL | 0.4 mL |
| CEM×174 (3×105) | 0.4 mL | 0.4 mL | 0.4 mL |
| Culture solution | - | - | 0.8 mL |

(3) The determination indexes for results:

1) The positive cell ratio of virus antigens% =(The fluorescence cells in the virus control holes %-The fluorescence cells in the drug holes %)÷The fluorescence cells in virus control holes %

2) Calculating the reduction of virus yield by log 10, and comparing the experimental holes and the virus control holes. If the virus tilter in the experimental holes reduced for one dilution, that is the virus yield reduced for 0.3 log 10, while if the experimental holes reduced for two dilutions, that is the virus yield reduced for 0.6 log 10, and others are analogized. For example: the virus tilter in the experimental holes is 1:320, the virus tilter in the virus control holes is 1:2560, then the virus yield in the experimental holes is determined to reduce for 0.9 log 10.

3) Cytopathic effect (CPE): fusing cells, it is + with at most 25% per field; it is ++ with at most 50% per filed; it is +++ with at most 75% per filed; and it is ++++ with more than 75% per field.

4) The result determination

To determine by taking the inhibition rate of the fluorescence positive cells and the reduction of the virus yield as mutual references, while the CPE extent is just for reference.

No inhibition: the inhibition rate of fluorescence positive cells is less than 30%, while the reduction of the virus yield is less than 0.6 log 10.

Mild inhibition: the inhibition rate of fluorescence positive cells is at least 30%, while the reduction of the virus yield is at least 0.6 log 10.

Moderate inhibition: the inhibition rate of fluorescence positive cells is at least 50%, while the reduction of the virus yield is at least 1.2 log 10.

High inhibition: the inhibition rate of fluorescence positive cells is at least 60%, while the reduction of the virus yield is at least 2.1 log 10.

III. Experimental Results (See Tab. 3.)

**Table 3 The experimental results for resisting SIV virus in vitro with HHA2 and HH2B**

| Drug | Dilution | Real concentration (µg) | Inhibition rate of virus antigen cells % | Reduction of virus tilter (log₁₀) | CPE | Results |
|---|---|---|---|---|---|---|
| HH2A | 1:40 | - | 51.8 | 0.6 | ++ and +++ | Light inhibition |
| | 1:160 | - | 27.8 | 0.6 | ++++ | No inhibition |
| HH2B | 1:10 | - | 49.9 | 1.21 | +++ | Moderate inhibition |
| | 1:40 | - | 17.4 | 0.6 | ++++ | No inhibition |
| AZT | | 2 µM | 89.3 | 2.71 | + | High inhibition |

Seen from the results in Table 3, the target sample HH2A had mild inhibition effect on SIV in vitro after forty times dilution, but it lost the anti-SIV effect when it was diluted four times to 1:160. HH2B with the concentration of 1:10 had mild toxicity effect on CEM×174 cells, also this concentration showed approximately moderate anti-SIV effect, but this medical solution could not bear the dilution, and lost anti-SIV effect when its concentration ratio was 1:40.

Conclusions: Based on the experiments above, as long as the proportion of Solidago virgaurea and water was reduced from 1:120, or extraction method was changed, for example, using the presently most advanced supercritical CO₂ fluid extraction technology, then extractions with high concentrations were gained, the ideal anti-AIDS virus effect satisfying the practice can be achieved, thus realizing the objective to treat and prevent AIDS.

The following examples are used to further explain this invention.

Example 1

100 g of Solidago virgaurea and 5,000 mL of water were transferred into the extractor, after boiling for 10 minutes, 4,800 mL of the herbal stock solution was obtained. Oral liquids or decoction pieces could be prepared according to the conventional method after filtering and concentrating.

Example 2

100 g of Solidago virgaurea and 10,000 mL of water were transferred into the extractor, after boiling for 25 minutes, 9,500 ml of the herbal stock solution was obtained. Oral liquids or decoction pieces could be prepared according to the conventional method after filtering and concentrating.

Example 3

90 g of Solidago virgaurea, 10 g of glycyrrhiza and 5,000 mL of water were transferred into the extractor, after boiling for 30 minutes and filtering, 4400 ml of the herbal stock solution was obtained.

Example 4

50 g of Solidago virgaurea, 25 g of honeysuckle, and 25 g of radix isatidis and 5,000 mL of water were transferred into the extractor, after boiling for 30 minutes and filtering, 4,400 mL of the herbal stock solution was obtained.

Example 5

100 g of Solidago virgaurea was used to prepare granules, capsules or decoction pieces in this invention after ultrafine milling according to the conventional method.

Example 6

90 g of Solidago virgaurea and 10 g of glycyrrhiza were used to prepare granules, capsules or decoction pieces in this invention after ultrafine milling according to the conventional method.

Example 7

50 g of Solidago virgaurea, 25 g of honeysuckle and 25 g of radix isatidis were used to prepare granules, capsules or decoction pieces in this invention after ultrafine milling according to the conventional method.

Example 8

100 g of Solidago virgaurea was used to prepare injections, granules, sprays, capsules or dropping pills in this invention after supercritical CO₂ fluid extraction according to the conventional method.

Example 9

90 g of Solidago virgaurea and 10 g of glycyrrhiza were used to prepare injection, granules, sprays, capsules or dropping pills in this invention after supercritical CO₂ fluidextraction according to the conventional method.

Example 10

50 g of Solidago virgaurea, 25 g of honeysuckle, and 25 g of radix isatidis were used to prepare injection, granules, sprays, capsules or dropping pills in this invention after supercritical CO₂ fluid extraction according to the conventional method.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. Use of Solidago virgaurea in the preparation of a medicament for treatment or prevention of a virus, wherein said medicament comprises 50-100% Solidago virgaurea by weight.

2. The use of Solidago virgaurea in the preparation of a medicament for treatment or prevention of a virus according to claim 1, wherein said virus is H5N1 avian influenza virus or influenza virus.

3. The use of Solidago virgaurea in the preparation of a medicament for treatment or prevention of a virus according to claim 1, wherein said virus is HIV.

4. The use of Solidago virgaurea in the preparation of a medicament for treatment or prevention of a virus according to claim 1, wherein said medicament comprises as the sole active ingredient Solidago virgaurea, said Solidago virgaurea being mixed with water in a weight ratio of 1:50-100 in an extractor, boiled for 10-30 minutes, filtered and purified with conventional methods to obtain an herbal stock solution.

5. The use of Solidago virgaurea in the preparation of a medicament for treatment or prevention of a virus according to claim 1, wherein said medicament comprises as the sole active ingredient Solidago virgaurea, said Solidago virgaurea being mixed with alcohol in a weight ratio of 1:50-100 in an extractor, boiled for 10-30 minutes, filtered and purified with conventional methods to obtain an herbal stock solution.

6. The use of Solidago virgaurea in the preparation of a medicament for treatment or prevention of a virus according to claim 1, wherein said medicament is provided in the form of an oral liquid, a granule, a decoction, a capsule, spray, a pill, an injection solution, or freeze-dried powder.

7. The use of Solidago virgaurea in the preparation of a medicament for treatment or prevention of a virus of claim 1, wherein said Solidago virgaurea is the entire Solidago virgaurea plant.

8. The use of Solidago virgaurea in the preparation of a medicament for treatment or prevention of a virus according to claim 1, wherein the active ingredients of said medicament comprise by weight 50-90% Solidago virgaurea and 10-50% glycyrrhiza.

9. The use of Solidago virgaurea in the preparation of a medicament for treatment or prevention of a virus according to claim 1, wherein the active ingredients of said medicament comprises by weight 50-90% Solidago virgaurea, 5-25% honeysuckle, and 5-25% radix isatidis.

10. Use of Solidago virgaurea in the preparation of a medicament for treatment or prevention of a virus, wherein said medicament is provided in form of a granule, a capsule or a herb decoction piece prepared through micron-milling technology, or in form of tea packages prepared through milling by common disintegrator, and the weight ratio of Solidago virgaurea with respect to the entire composition is 50-100%.

11. The use of Solidago virgaurea in the preparation of a medicament for treatment or prevention of a virus according to claim 10, the active ingredients of said medicament comprise by weight.50-90% Solidago virgaurea and 10-50% glycyrrhiza

12. The use of Solidago virgaurea in the preparation of a medicament for treatment and prevention of a virus of claim 10, wherein the active ingredients of said medicament comprises 50-90% Solidago virgaurea, 5-25% honeysuckle, and 5-25% radix isatidis by weight.

13. The use of Solidago virgaurea in the preparation of a medicament for treatment and prevention of a virus of claim 1, wherein said medicament is provided in form of an injection preparation, a freeze dried powder, a decoction, an oral liquid, a granule, spray, a capsule, or a pill; said Solidago virgaurea is provided in form of an extract by supercritical CO₂, and the weight ratio of Solidago virgaurea with respect to the entire composition is 50-100%.
